# EUROPEAN PATENT APPLICATION

(11) **EP 4 671 756 A1**
(43) Date of publication of application: **31.12.2025**
(21) Application number: 24184161.8
(22) Date of filing: 25.06.2024
(51) Int. Cl.: G01N 33/50, G01N 33/554

(54) **METHOD AND SYSTEM FOR CONSTRUCTING SENSOR SURFACE AND USAGE THEREOF**

(71) Applicant: Stichting IMEC Nederland, 6708 WH Wageningen (NL)
(72) Inventor: Tibbe, Arjan, 7425 PG Deventer (NL); Karcz, Adriana, 5629 NE Eindhoven (NL)
(74) Representative: Körfer, Thomas

(57) **Abstract**

A method (100) for constructing a sensor surface, the method comprises a step of providing (101) a buffer fluid comprising at least one sensing particle. In addition, the method further comprises a step of providing (102) a membrane comprising a plurality of pores, each of the plurality of pores has a pore size smaller than the sensing particle. In addition, the method comprises a step of the arranging (103) the membrane in relation to the buffer fluid such that a first surface of the membrane being in fluidic contact with the buffer fluid. Moreover, the method comprises a step of pushing (104) the buffer fluid through the membrane to immobilize the sensing particle on the first surface of the membrane.

## Description

The invention relates to the construction of sensor surfaces, especially whole-cell biosensor surfaces, and further to the use of said sensor surfaces, especially in the form of whole-cell biosensors, for detecting and/or measuring biological and/or chemical activities.

Generally, whole-cell sensors can sense compounds of interests, i.e., analytes, in complex fluids and matrices that contain a variety of molecules and particles, e.g., the real time detection and concentration measurements of specific proteins inside bioreactors. The general concept of whole-cell biosensors is that living cells convert inputs which are otherwise difficult to measure, e.g., metabolites, chemicals, cytokines, hormones, as well as other compounds that cannot be measured with existing biosensors, into the measurable physical parameters.

For example, the presence of a certain protein can be specifically detected by the cell that, in turn, generates a physical signal, such as light or charge, which can be detected by conventional detectors. Ideally, the instructions for the cell sensing, processing and actuation abilities are embedded in the DNA of the cell using different biotechnological, biosynthetic, and genetic engineering methods.

The use of porous membranes is commonly known in cellular co-culture systems. For example, the document EP 2 636 729 A1 discloses a microporous membrane suited to cell culture, which is placed in a microwell plate in order for cells to be cultured on the membrane in the microwell plate. However, unlike in the co-culture systems and in the field of genetically engineered sensing, avoidance of mixing of said genetically engineered sensing with other cells needs to be taken into consideration.

Current methodologies for building biosensor surfaces mostly rely on printing technologies in combination with surface chemistries to fix and mount the biosensors on the sensor surface. Additionally, in order to enable successful printing, the fluids in which the biosensors reside need to match the printer requirements as well as the properties of the material of the sensor surface, such as hydrophobicity among others.

Accordingly, an object of the invention is to provide a method and a system for constructing a sensor surface in a simplified manner in order to overcome the above-mentioned limitations. Another object is to provide a sensor for detecting and/or measuring biological and/or chemical activities in a simplified and cost-effective manner.

These and other objects are solved by the features of the first independent claim for the method, by the features of the second independent claims for the system, and by the features of the third independent claim for the sensor. The dependent claims contain further developments.

According to a first aspect of the invention, a method for constructing a sensor surface is provided. The method comprises a step of providing a buffer fluid comprising at least one sensing particle. In addition, the method further comprises a step of providing a membrane comprising a plurality of pores, each of the plurality of pores has a pore size smaller than the sensing particle.

In addition, the method comprises a step of arranging the membrane in relation to the buffer fluid such that a first surface of the membrane being in fluidic contact with the buffer fluid. Moreover, the method comprises a step of pushing the buffer fluid through the membrane to immobilize and/or to concentrate the sensing particle on the first surface of the membrane.

Therefore, a physical confinement is facilitated by separating the sensing particles, especially whole-cell biosensor cells, from the sample fluid, thereby forming a sensor surface, especially a whole-cell biosensor surface, in a simplified manner.

Advantageously, by using the porous membrane to immobilize the sensing particles to form the sensor surface, the sensing particles can be suspended in any type of fluid independently on the surface properties or printer requirements. Furthermore, the sensing particles can be concentrated on the sensing surface. Moreover, the sensing particles cannot escape from the sensing surface, which eliminates any further application of chemical crosslinking and surface chemistry.

Preferably, the sensing particle is configured to produce light and/or to change color and/or to change charge and/or to produce gases and/or to change in size and/or to produce at least one measurable change in reaction to sample analytes. For instance, the sensing particles may be hydrogel beads synthesized with a glucose sensitive dye designed to sense glucose.

Preferably, the sensing particle comprises at least one whole-cell eukaryotic cell or prokaryotic cell, or at least one non-whole cell particle containing one or more whole-cell components, or at least one hydrogel particle, or at least one polystyrene particle, or at least one ceramic particle, or a combination thereof.

It is to be noted that the sensing particle is not limited to the above-mentioned hydrogel, polystyrene, and ceramic materials. Other types of suitable materials can be used to form or to create the sensing particle.

Preferably, the membrane comprises the plurality of pores having a pore size between 0.1 to 0.5 microns, preferably a pore size between 0.2 to 0.3 microns, more preferably a pore size between 0.20 to 0.25 microns.

According to a second aspect of the invention, a system for constructing a sensor surface is provided. The system comprises at least one pumping arrangement comprising a buffer fluid comprising at least one sensing particle, the pumping arrangement being configured to push the buffer fluid through an opening of the pumping arrangement. In addition, the system comprises at least one filter arrangement comprising a membrane comprising a plurality of pores, each of the plurality of pores having a pore size smaller than the sensing particle.

In this regard, the filter arrangement is detachably attached to the opening of the pumping arrangement such that a first surface of the membrane being in fluidic contact with the buffer fluid. Furthermore, the membrane is configured to immobilize and/or to concentrate the sensing particle on the first surface while the buffer fluid being pushed through the membrane by the pumping arrangement.

Therefore, a physical confinement is facilitated by separating the sensing particles, especially whole-cell biosensor cells, from the sample fluid, thereby forming a sensor surface, especially a whole-cell biosensor surface, in a simplified manner.

Preferably, the system comprises at least one further pumping arrangement comprising a further buffer fluid comprising at least one further sensing particle. In addition, the system comprises at least one further filter arrangement comprising a further membrane comprising a plurality of pores, each of the plurality of pores having a pore size smaller than the further sensing particle.

In this regard, the further membrane is configured to immobilize and/or to concentrate the further sensing particle on a first surface of the further membrane while the further buffer fluid being pushed through the further membrane by the further pumping arrangement.

Advantageously, a plurality of sensor surfaces, especially whole-cell biosensor surfaces, can be formed in a simplified manner, which can perform parallel sensing of a plurality of analytes, respectively.

Preferably, the membrane and/or the further membrane comprise the plurality of pores having a pore size between 0.1 to 0.5 microns, preferably a pore size between 0.2 to 0.3 microns, more preferably a pore size between 0.20 to 0.25 microns.

Preferably, the pumping arrangement and/or the further pumping arrangement are syringes or have a syringe-like configuration. Advantageously, for example, the fluid delivery mechanism can be realized in a simplified manner with a high precision and control.

Preferably, the filter arrangement and/or the further filter arrangement are syringe filters or filters having components of syringe. For example, the filter arrangement may comprise or be a syringe filter with a pore size of 0.22 microns. Alternatively, the filter arrangement may comprise or be a syringe filter with a pore size of 0.45 microns.

According to a third aspect of the invention, a sensor is provided. The sensor comprises at least one filter arrangement comprising a membrane, and at least one sensing particle immobilized on a first surface of the membrane, especially according to the method of the first aspect of the invention. The sensor further comprises a readout module being arranged in relation to the first surface of the membrane.

In this regard, the filter arrangement is configured to pass analytes onto a second surface of the membrane opposite to the first surface in order for the analytes to diffuse through the membrane. Furthermore, the readout module is configured to detect a property of the sensing particle in reaction to the analytes diffused through the membrane.

Therefore, an application of the sensor surface, i.e., the sensor surface constructed by separating the sensing particles from the sample fluid, is provided by means of sensor particles, especially whole-cell biosensors, which may detect and/or measure biological and/or chemical activities in reaction to the analytes.

Preferably, the filter arrangement is detachably attached to the readout module. Advantageously, for example, the readout module can be detached from one filter arrangement, and may be re-used for another filter arrangement.

Preferably, the readout module comprises an optical detector configured to detect an optical property of the sensing particle in reaction to the analytes diffused through the membrane.

Alternatively, the readout module comprises an electrical signal detector configured to detect an electrical property of the sensing particle in reaction to the analytes diffused through the membrane.

Alternatively, the readout module comprises a detector unit configured to simultaneously detect an optical property and an electrical property of the sensing particle in reaction to the analytes diffused through the membrane.

Preferably, the filter arrangement comprises a first channel being in fluidic contact with the first surface of the membrane, and a second channel being in fluidic contact with the second surface of the membrane. In this regard, the first channel is configured to confine the sensing particle immobilized on the first surface of the membrane and/or the analytes diffused through the membrane.

Advantageously, for example, a fluidic isolation between the filter arrangement and the readout module is facilitated. Furthermore, the second channel is configured to pass the analytes onto the second surface of the membrane for the analytes to diffuse through the membrane.

Preferably, the filter arrangement and/or the detector module are sterilizable. Advantageously, for example, the sensor can be effectively used in bio-controlled environments.

It is to be noted that the system according to the second aspect corresponds to the method according to the first aspect and its implementation forms. It is further to be noted that the elements and/or components of the sensor according to the third aspect may have corresponding implementation forms of the analogous elements and/or components according to the method of the first aspect and/or the system of the second aspect.

Exemplary embodiments of the invention are now further explained with respect to the drawings by way of example only, and not for limitation. In the drawings:
- Fig. 1: shows an exemplary embodiment of the method according to the first aspect of the invention;
- Fig. 2: shows a first exemplary embodiment of the system according to the second aspect of the invention;
- Fig. 3: shows a second exemplary embodiment of the system according to the second aspect of the invention;
- Fig. 4: shows a third exemplary embodiment of the system according to the second aspect of the invention;
- Fig. 5: shows a first exemplary embodiment of the sensor according to the third aspect of the invention;
- Fig. 6: shows a second exemplary embodiment of the sensor according to the third aspect of the invention;
- Fig. 7: shows a first exemplary embodiment of the readout module according to the third aspect of the invention;
- Fig. 8: shows a second exemplary embodiment of the readout module according to the third aspect of the invention; and
- Fig. 9: shows a third exemplary embodiment of the readout module according to the third aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

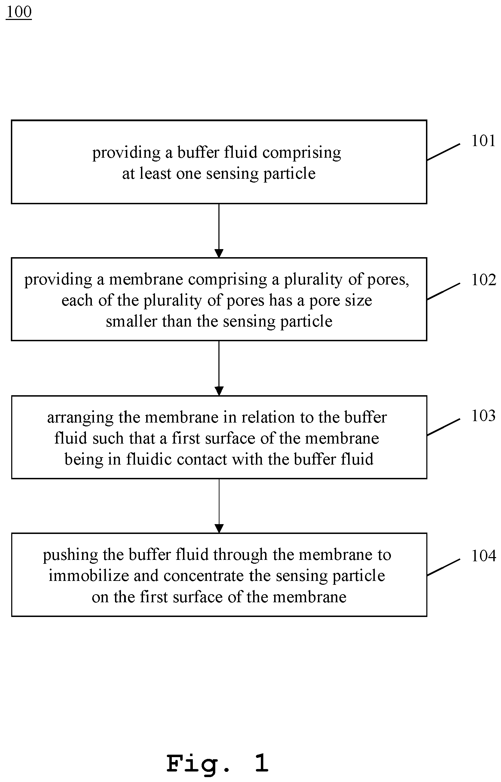

In Fig. 1, an exemplary embodiment of the method 100 according to the first aspect of the invention is illustrated. In a first step 101, a buffer fluid comprising at least one sensing particle is provided. In a second step 102, a membrane comprising a plurality of pores is provided, where each pore of the plurality of pores has a pore size smaller than the sensing particle.

In a third step 103, the membrane is arranged in relation to the buffer fluid such that a first surface of the membrane being in fluidic contact with the buffer fluid. In a fourth step 104, the buffer fluid is pushed through the membrane to immobilize and concentrate the sensing particle on the first surface of the membrane.

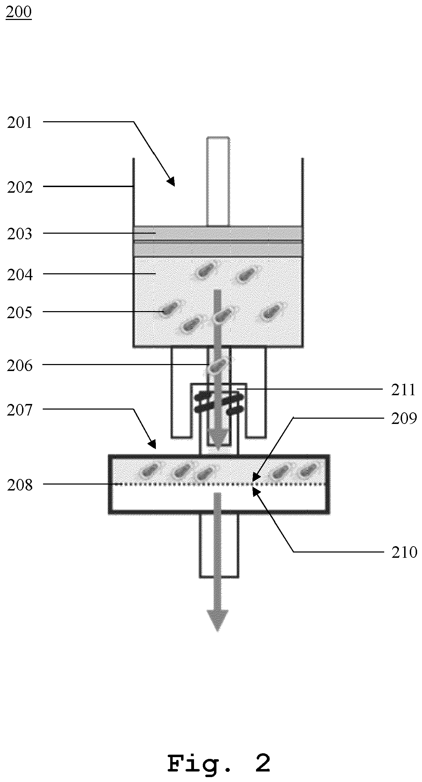

In Fig. 2, a first exemplary embodiment of the system 200 according to the second aspect of the invention is illustrated. The system 200 may comprise a pumping arrangement 201, especially a syringe, comprising a barrel 202, a plunger 203 that is tightly fitted to the inside of the barrel 202, and an opening 206. The plunger 203 can be linearly pushed along the inside of the barrel 202 towards the opening 206.

The pumping arrangement 201 may contain buffer fluid 204 comprising sensing particles or sensing cells 205, especially confined inside of the barrel 202 between the plunger 203 and the opening 206.

The system 200 may further comprise a filter arrangement 207, especially a syringe filter, comprising a porous membrane 208, especially a microporous membrane, with pore size smaller than the size of the sensing particles 205 in the buffer fluid 204. The filter arrangement 207 may be detachably attached to the pumping arrangement 201 via a locking mechanism 211, such as a luer-taper or a luer-lock fluidic fitting, such that a first surface 209 of the porous membrane 208 being in fluidic contact with the opening 206.

As such, the buffer fluid 204 may be pushed by the pumping arrangement 201, especially by the plunger 203, through the opening 206, and further through the porous membrane 208. Since the porous membrane 208 has pore size smaller than the sensing particles 205, only the buffer fluid 204 can be passed through the porous membrane 208 towards a second surface 210 of the porous membrane 208, leaving behind the sensing particles 205 immobilized on the first surface 209.

The bold arrow shows the flow direction of the buffer fluid 204 through the opening 206 and further through the porous membrane 208, especially due to the force applied by the plunger 203 of the pumping arrangement 201. As such, the filter arrangement 207 can be detached from the pumping arrangement 201, and the first surface 209 of the porous membrane 208 with the immobilized sensing particles 205 can be used as a sensing surface, especially as a biosensor surface.

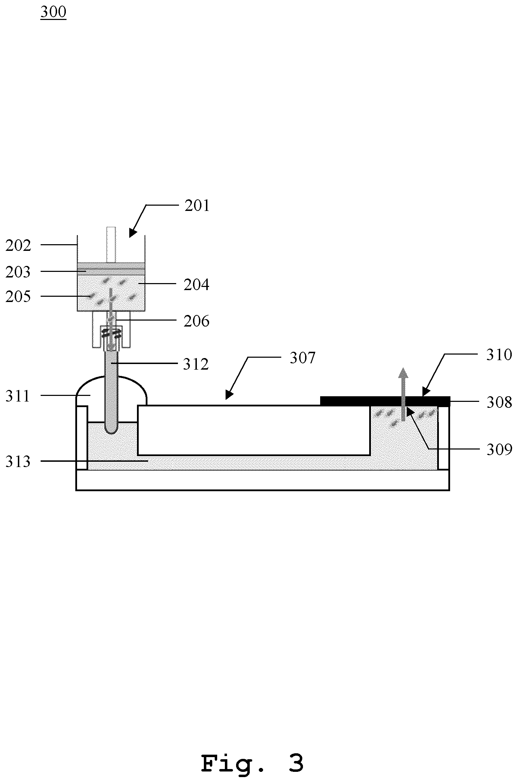

In Fig. 3, a second exemplary embodiment of the system 300 according to the second aspect of the invention is illustrated. The system 300 may comprise a pumping arrangement 201 analogous to the system 200, i.e., comprising a barrel 202, a plunger 203, and an opening 206, where the barrel may contain a buffer fluid 204 comprising sensing particles 205.

The system 300 may further comprise a filter arrangement 307 comprising a porous membrane 308, especially a microporous membrane, with pore size smaller than the size of the sensing particles 205 in the buffer fluid 204. The filter arrangement 307 may comprise a fluidic channel 313 being in fluidic contact with a first surface 309 of the porous membrane 308.

For instance, the filter arrangement 307 may be detachably attached to the pumping arrangement 201 via a locking mechanism, such as a rubber cap or stopper 311 arranged at an opening of the fluidic channel 313 and a needle 312 arranged at the opening 206 of the pumping arrangement 201.

Therefore, the buffer fluid 204 can be pushed through the rubber cap or stopper into the fluidic channel 313 and the pumping arrangement 201 can be detached easily when needed. Accordingly, the first surface 209 of the porous membrane 208 being in fluidic contact with the opening 206 of the pumping arrangement 201 via the fluidic channel 313.

As such, the buffer fluid 204 may be pushed by the pumping arrangement 201, especially by the plunger 203, through the opening 206, along the fluidic channel 313, and further through the porous membrane 308. Since the porous membrane 308 has pore size smaller than the sensing particles 205, only the buffer fluid 204 can be passed through the porous membrane 308 towards a second surface 310 of the porous membrane 308, leaving behind the sensing particles 205 immobilized on the first surface 309.

The bold arrow shows the flow direction of the buffer fluid 204 through the opening 206 and further through the porous membrane 308, especially due to the force applied by the plunger 203 of the pumping arrangement 201. As such, the filter arrangement 307 can be detached from the pumping arrangement 201, and the first surface 309 of the porous membrane 308 with the immobilized sensing particles 205 can be used as a sensing surface, especially as a biosensor surface.

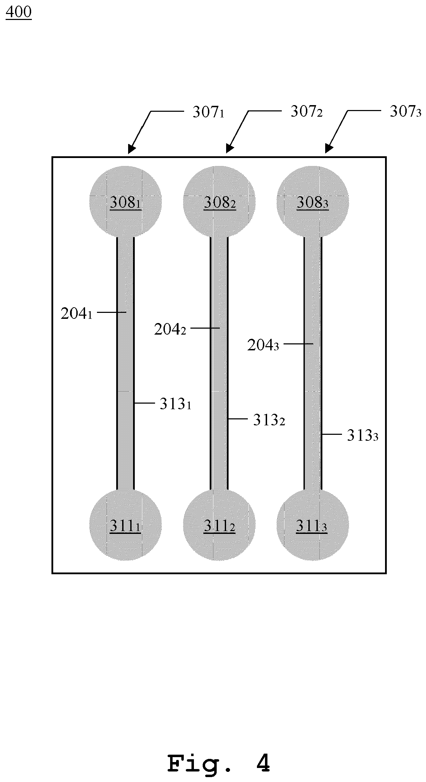

In Fig. 4, a third exemplary embodiment of the system 400 according to the second aspect of the invention is illustrated. The system 400 comprises a plurality of filter arrangements 307, exemplary a first filter arrangement 307₁, a second filter arrangement 307₂, and a third filter arrangement 307₃. However, the number of the filter arrangements can be more than or less than three, as illustrated herein. For example, each of the filter arrangements 307₁-307₃ may correspond to the filter arrangement 307 of the system 300.

For instance, the first filter arrangement 307₁ may comprise a porous membrane 308₁ with pore size smaller than the size of the sensing particles 205 in a first buffer fluid 204₁, a fluidic channel 313₁ being in fluidic contact with a first surface of the porous membrane 308₁, and a locking mechanism 311₁. In this regard, the first buffer fluid 204₁ may be pushed by a first pumping arrangement (not shown) along the fluidic channel 313₁, and further through the porous membrane 308₁, thereby immobilizing the sensing particles 205 on the first surface of the porous membrane 308₁.

Similarly, the second filter arrangement 307₂ may comprise a porous membrane 308₂ with pore size smaller than the size of the sensing particles 205 in a second buffer fluid 204₂, a fluidic channel 313₂ being in fluidic contact with a first surface of the porous membrane 308₂, and a locking mechanism 311₂. In this regard, the second buffer fluid 204₂ may be pushed by a second pumping arrangement (not shown) along the fluidic channel 313₂, and further through the porous membrane 308₂, thereby immobilizing the sensing particles 205 on the first surface of the porous membrane 308₂.

Analogously, the third filter arrangement 307₃ may comprise a porous membrane 308₃ with pore size smaller than the size of the sensing particles 205 in a third buffer fluid 204₃, a fluidic channel 313₃ being in fluidic contact with a first surface of the porous membrane 308₃, and a locking mechanism 311₃. In this regard, the third buffer fluid 204₃ may be pushed by a third pumping arrangement (not shown) along the fluidic channel 313₃, and further through the porous membrane 308₃, thereby immobilizing the sensing particles 205 on the first surface of the porous membrane 308₃.

It is to be noted, each of the first pumping arrangement, the second pumping arrangement, and the third pumping arrangement may correspond to the pumping arrangement 201 of the system 200. Furthermore, each of the first buffer fluid 204₁, the second buffer fluid 204₂, and the third buffer fluid 204₃ may correspond to the buffer fluid 204 of the system 200.

Alternatively, the first buffer fluid 204₁, the second buffer fluid 204₂, and the third buffer fluid 204₃ may be different from each other, i.e., comprising different sensing particles that may produce different measurable changes in reaction to different sample analytes.

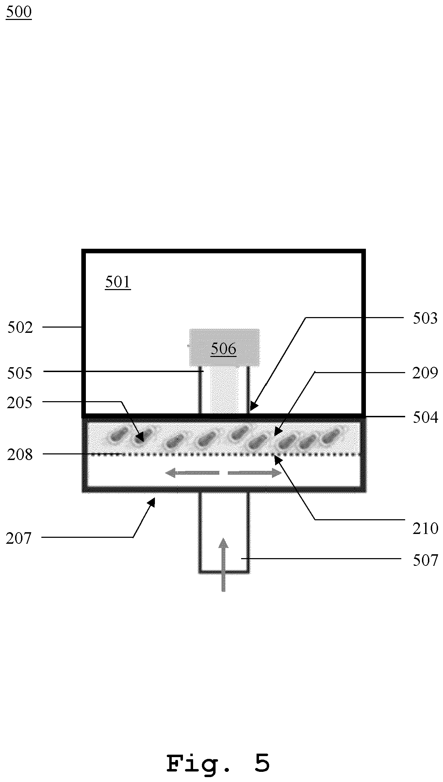

In Fig. 5, a first exemplary embodiment of the sensor 500 according to the third aspect of the invention is illustrated. The sensor 500 may comprise a filter arrangement 207, which may correspond to the filter arrangement 207 of the system 200, where the sensing particles 205 are immobilized on the first surface 209 of the porous membrane 208.

The sensor 500 may further comprise a readout module 501 arranged in relation to the first surface 209 of the porous membrane 208. In this regard, the readout module 501 may comprise a housing 502, and an aperture 503 especially arranged at an attachment surface 504 of the housing 502.

For example, the filter arrangement 207 may comprise a first channel 505 being in fluidic contact with the first surface 209 of the porous membrane 208, and a second channel 507 being in fluidic contact with the second surface 210 of the porous membrane 208.

For instance, in order to attach the filter arrangement 207 to the readout module 501, the first channel 505 of the filter arrangement 207 may be positioned inside of the housing 502 of the readout module 501, especially through the aperture 503 at the attachment surface 504, and may be fixed at the attachment surface 504, e.g., using an adhesive substance. Therefore, the filter arrangement 207 can be detached from the readout module 501 if needed.

For example, in order to provide a fluidic isolation between the filter arrangement 207 and the readout module 501, the first channel 505 of the filter arrangement 207 may comprise a locking mechanism 506, e.g., a luer-lock, which may restrict the sensing particles 205 and any remaining buffer fluid 204 within the filter arrangement 207, especially in the first channel 505.

For instance, the second channel 507 of the filter arrangement 207 may allow sample fluids comprising compounds of interest, e.g., bioreactor sample fluids comprising analytes, to pass onto the second surface 210 of the porous membrane 208 for the analytes to be diffused through the porous membrane 208. The bold arrow shows the flow direction of a sample fluid through the second channel 507 of the filter arrangement 207 onto the second surface 210 of the porous membrane 208.

In this regard, the readout module 501 may detect a property of the sensing particles 205, e.g., one or more optical properties and/or one or more electrical properties of the sensing particles 205, in reaction to the analytes diffused through the porous membrane 208.

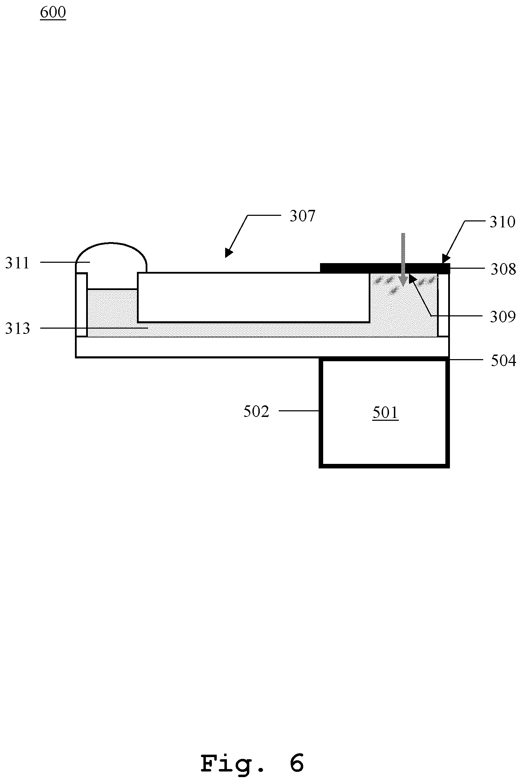

In Fig. 6, a second exemplary embodiment of the sensor 600 according to the third aspect of the invention is illustrated. The sensor 600 may comprise a filter arrangement 307, which may correspond to the filter arrangement 307 of the system 300, where the sensing particles 205 are immobilized on the first surface 309 of the porous membrane 308.

The sensor 600 may further comprise a readout module 501 arranged in relation to the first surface 309 of the porous membrane 308. The readout module 501 may correspond to the readout module 501 of the sensor 500. In order to attach the filter arrangement 307 to the readout module 501, the filter arrangement 307 may be fixed at the attachment surface 504 of the readout module 501, e.g., using an adhesive substance. Therefore, the filter arrangement 307 can be detached from the readout module 501 if needed.

For instance, the filter arrangement 307 may allow sample fluids comprising compounds of interest, e.g., bioreactor sample fluids comprising analytes, to pass onto the second surface 310 of the porous membrane 308 for the analytes to be diffused through the porous membrane 308. The bold arrow shows the diffusion of analytes from the second surface 310 to the first surface 309 through the porous membrane 308.

In this regard, the readout module 501 may detect a property of the sensing particles 205, e.g., one or more optical properties and/or one or more electrical properties of the sensing particles 205, in reaction to the analytes diffused through the porous membrane 308.

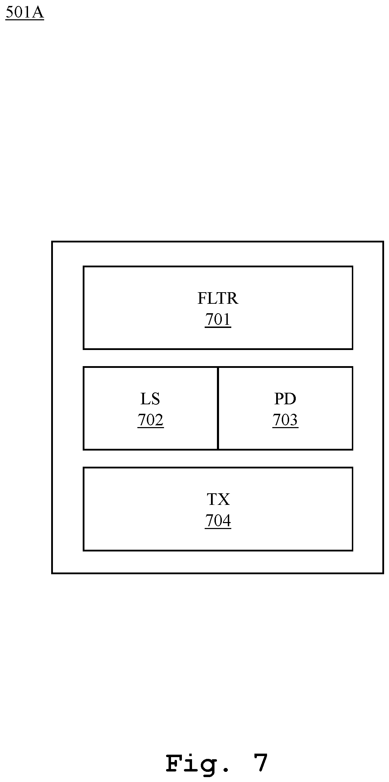

In Fig. 7, a first exemplary embodiment of the readout module 501A according to the third aspect of the invention is illustrated. The readout module 501A may comprise or be an optical detector and may detect an optical property of the sensing particle 205, e.g., a light signal produced and/or a change in color in reaction to the analytes diffused through the membrane 208, 308.

For example, the readout module 501A may comprise a filter or grating structure 701 configured for an excitation light signal of a predefined wavelength and further for an emission light signal of a predefined wavelength. The readout module 501A may further comprise a light source 702, such as a light-emitting diode, which may generate the excitation light signal to illuminate the sensing particle 205 especially through the filter structure 701, e.g., to achieve the excited state of the sensing particle 205.

The readout module 501A may further comprise a photodetector 703, such as a photodiode, which may receive the emission light signal from the sensing particle 205, especially through the filter structure 701. The readout module 501A may further comprise a transmitter 704 that may receive the optical measurements from the photodetector 703, e.g., the photodiode currents or voltages, and may transmit the measurements to an external device, especially wirelessly, for further pursing and/or processing of the measurement data.

For instance, the readout module 501A may additionally comprise a processor (not shown) that may perform preprocessing of the optical measurements before sending out the measurements through the transmitter 704. In addition, the readout module 501A may comprise a power source (not shown), e.g., a battery, in order to provide electric power to the components of the readout module 501A.

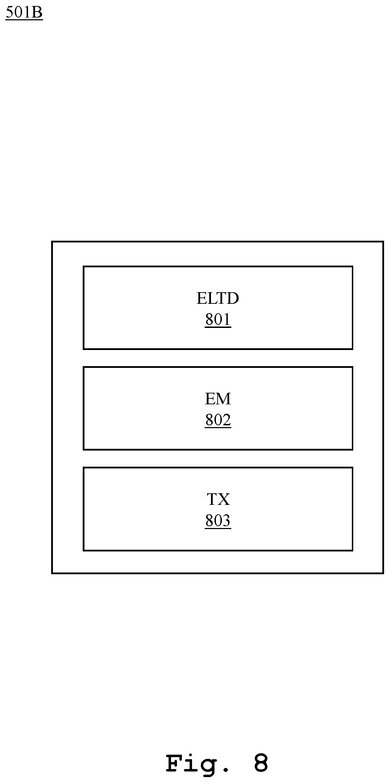

In Fig. 8, a second exemplary embodiment of the readout module 501B according to the third aspect of the invention is illustrated. The readout module 501A may comprise or be an electrical signal detector and may detect an electrical property of the sensing particle 205, e.g., the production and/or change in charge in reaction to the analytes diffused through the membrane 208, 308.

For example, the readout module 501B may comprise an electrode 801 configured to be in electrically coupled to the sensing particle 205. The detector 501B may further comprise an electrometer 802, such as a potentiometer, electrically coupled to the electrode 801. The electrometer 802 may measure an electrical signal produced by the sensing particle 205.

The detector 501B may further comprise a transmitter 803 that may receive the electrical measurements from the electrometer 802 and may transmit the measurements to an external device, especially wirelessly, for further pursing and/or processing of the measurement data.

For instance, the readout module 501B may additionally comprise a processor (not shown) that may perform preprocessing of the electrical measurements before sending out the measurements through the transmitter 803. In addition, the readout module 501B may comprise a power source (not shown), e.g., a battery, in order to provide electric power to the components of the readout module 501B.

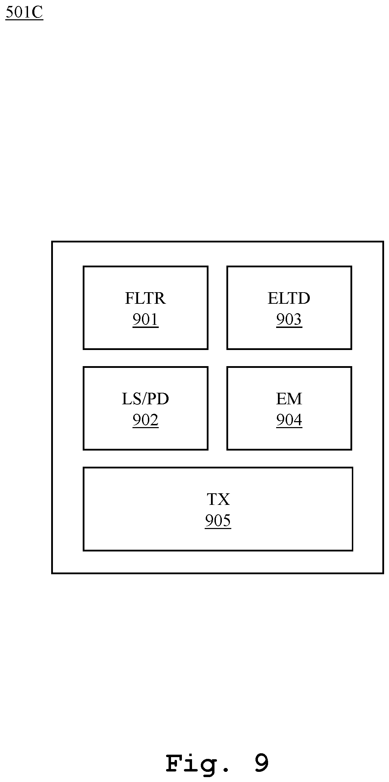

In Fig. 9, a third exemplary embodiment of the readout module 501C according to the third aspect of the invention is illustrated. The readout module 501A may comprise or be a detector unit or a hybrid detector and may detect an optical property of the sensing particle 205, e.g., a light signal produced and/or a change in color, as well as an electrical property of the sensing particle 205, e.g., the production and/or change in charge, especially in reaction to the analytes diffused through the membrane 208, 308.

For example, the readout module 501C may comprise a filter or grating structure 901 configured for an excitation light signal of a predefined wavelength and further for an emission light signal of a predefined wavelength. The readout module 501C may further comprise a light detection unit 902, such as comprising a light-emitting diode and a photodiode, which may generate the excitation light signal and may receive the emission light signal from the sensing particle 205, especially through the filter structure 901.

In addition, the readout module 501C may comprise an electrode 903 configured to be in electrically coupled to the sensing particle 205, and an electrometer 904, such as a potentiometer, electrically coupled to the electrode 903. The electrometer 904 may measure an electrical signal produced by the sensing particle 205.

The readout module 501C may further comprise a transmitter 905 that may receive the optical measurements from the light detection unit 902 and the electrical measurements from the electrometer 904, and may transmit the measurements to an external device, especially wirelessly, for further pursing and/or processing of the measurement data.

For instance, the readout module 501C may additionally comprise a processor (not shown) that may perform preprocessing of the optical measurements and the electrical measurements before sending out the measurements through the transmitter 905. In addition, the readout module 501C may comprise a power source (not shown), e.g., a battery, in order to provide electric power to the components of the readout module 501C.

It is important to note that, in the description as well as in the claims, the word "comprising" does not exclude other elements or steps and the indefinite article "a" or "an" does not exclude a plurality. A single element or other unit may fulfill the functions of several entities or items recited in the claims.

It should be understood that the term "and/or" used in the specification and the appended claims of this application refers to any combination and all possible combinations of one or more associated listed items, and includes these combinations. Moreover, the disclosure with regard to any of the aspects is also relevant with regard to the other aspects of the disclosure.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A method (100) for constructing a sensor surface, the method comprises:
providing (101) a buffer fluid comprising at least one sensing particle,
providing (102) a membrane comprising a plurality of pores, each of the plurality of pores has a pore size smaller than the sensing particle,
arranging (103) the membrane in relation to the buffer fluid such that a first surface of the membrane being in fluidic contact with the buffer fluid, and
pushing (104) the buffer fluid through the membrane to immobilize the sensing particle on the first surface of the membrane.

2. The method according to claim 1,
wherein the sensing particle is configured to produce light and/or to change color and/or to change charge and/or to produce gases and/or to change in size and/or to produce at least one measurable change in reaction to sample analytes.

3. The method according to claim 1 or 2,
wherein the sensing particle comprises at least one whole-cell eukaryotic cell or prokaryotic cell, or at least one non-whole cell particle containing one or more whole-cell components, or at least one hydrogel particle, or at least one polystyrene particle, or at least one ceramic particle, or a combination thereof.

4. The method according to any of claims 1 to 3,
wherein the membrane comprises the plurality of pores having a pore size between 0.1 to 0.5 microns, preferably a pore size between 0.2 to 0.3 microns, more preferably a pore size between 0.20 to 0.25 microns.

5. A system (200, 300, 400) for constructing a sensor surface, the system comprises:
at least one pumping arrangement (201) comprising a buffer fluid (204) comprising at least one sensing particle (205), the pumping arrangement (201) being configured to push the buffer fluid (204) through an opening (206) of the pumping arrangement (201), and
at least one filter arrangement (207, 307) comprising a membrane (208, 308) comprising a plurality of pores, each of the plurality of pores having a pore size smaller than the sensing particle (205),
wherein the filter arrangement (207, 307) is detachably attached to the opening (206) of the pumping arrangement (201) such that a first surface (209, 309) of the membrane (208, 308) being in fluidic contact with the buffer fluid (204), and
wherein the membrane (208, 308) is configured to immobilize the sensing particle (205) on the first surface (209, 309) while the buffer fluid (204) being pushed through the membrane (208, 308) by the pumping arrangement (201).

6. The system according to claim 5,
wherein the system comprises:
at least one further pumping arrangement comprising a further buffer fluid (204₁-204₃) comprising at least one further sensing particle, and
at least one further filter arrangement (307₁-307₃) comprising a further membrane (308₁-308₃) comprising a plurality of pores, each of the plurality of pores having a pore size smaller than the further sensing particle,
wherein the further membrane (308₁-308₃) is configured to immobilize the further sensing particle on a first surface of the further membrane (308₁-308₃) while the further buffer fluid (204₁-204₃) being pushed through the further membrane (308₁-308₃) by the further pumping arrangement.

7. The system according to claim 5 or 6,
wherein the membrane (208, 308) and/or the further membrane (308₁-308₃) comprise the plurality of pores having a pore size between 0.1 to 0.5 microns, preferably a pore size between 0.2 to 0.3 microns, more preferably a pore size between 0.20 to 0.25 microns.

8. The system according to any of claims 5 to 7,
wherein the pumping arrangement (201) and/or the further pumping arrangement are syringes or have a syringe-like configuration, and/or
wherein the filter arrangement (207, 307) and/or the further filter arrangement (307₁-307₃) are syringe filters or filters having components of syringe.

9. A sensor (500, 600) comprising:
at least one filter arrangement (207, 307) comprising a membrane (208, 308), and at least one sensing particle (205) immobilized on a first surface (209, 309) of the membrane (208, 308), especially according to the method (100) of claims 1 to 4, and
a readout module (501) being arranged in relation to the first surface (209, 309) of the membrane (208, 308), wherein the filter arrangement (207, 307) is configured to pass analytes onto a second surface (210, 310) of the membrane (208, 308) opposite to the first surface (209, 309) in order for the analytes to diffuse through the membrane (208, 308), and
wherein the readout module (501) is configured to detect a property of the sensing particle (205) in reaction to the analytes diffused through the membrane (208, 308).

10. The sensor according to claim 9,
wherein the filter arrangement (207, 307) is detachably attached to the readout module (501).

11. The sensor according to claim 9 or 10,
wherein the readout module (501) comprises an optical detector (501A) configured to detect an optical property of the sensing particle (205) in reaction to the analytes diffused through the membrane (208, 308).

12. The sensor according to claim 9 or 10,
wherein the readout module (501) comprises an electrical signal detector (501B) configured to detect an electrical property of the sensing particle (205) in reaction to the analytes diffused through the membrane (208, 308).

13. The sensor according to claim 9 or 10,
wherein the readout module (501) comprises a detector unit (501C) configured to simultaneously detect an optical property and an electrical property of the sensing particle (205) in reaction to the analytes diffused through the membrane (208, 308).

14. The sensor according to any of claims 9 to 13,
wherein the filter arrangement (207, 307) comprises a first channel (505, 313) being in fluidic contact with the first surface (209, 309) of the membrane (208, 308), and a second channel (507) being in fluidic contact with the second surface (210, 310) of the membrane (208, 308),
wherein the first channel (505, 313) is configured to confine the sensing particle (205) immobilized on the first surface (209, 309) of the membrane (208, 308) and/or the analytes diffused through the membrane (208, 308), and
wherein the second channel (507) is configured to pass the analytes onto the second surface (210, 310) of the membrane (208, 308) for the analytes to diffuse through the membrane (208, 308).

15. The sensor according to any of claims 9 to 14,
wherein the filter arrangement (207, 307) and/or the detector module (501) are sterilizable.
